(19) Europäisches Patentamt
European Patent Office
Office européen des brevets

(11) **EP 4 700 005 A1**

(12) **EUROPÄISCHE PATENTANMELDUNG**

(43) Veröffentlichungstag:
**25.02.2026 Patentblatt 2026/09**

(21) Anmeldenummer: **24020273.9**

(22) Anmeldetag: **22.08.2024**

(51) Internationale Patentklassifikation (IPC):
***C07C 29/151*** *(2006.01)*

(52) Gemeinsame Patentklassifikation (CPC):
(C-Sets verfügbar)
**C07C 29/151** (Forts.)

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**GE KH MA MD TN**

(71) Anmelder: **Linde GmbH**
**82049 Pullach (DE)**

(72) Erfinder:
- **Dillig, Marius**
  **82049 Pullach (DE)**
- **Heinzel, Albrecht**
  **82049 Pullach (DE)**
- **Schwarzhuber, Josef**
  **82049 Pullach (DE)**
- **Korn, Wibke**
  **82049 Pullach (DE)**
- **Reinke, Michael**
  **82049 Pullach (DE)**

(74) Vertreter: **Fischer, Werner**
**Linde GmbH**
**Intellectual Property EMEA**
**Dr.-Carl-von-Linde-Straße 6-14**
**82049 Pullach (DE)**

(54) **VERFAHREN UND ANLAGE ZUR HERSTELLUNG VON METHANOL**

(57) Die Erfindung betrifft ein Verfahren sowie eine Anlage zur Erzeugung von Methanol, bei dem Wasserstoff (101a) in einem ersten Betriebsmodus mit einem oberhalb eines Schwellwerts liegenden Mengenstrom bereitgestellt und zusammen mit Kohlenstoffdioxid (101b) als Frischeinsatz (101) in einen Synthesekreislauf eingeleitet wird, um einen Wasserstoff und Kohlenstoffdioxid umfassenden Syntheseeinsatz (103) zu bilden, aus dem in einem Methanol-Synthesereaktor (40) ein Wasserstoff, Kohlendioxid, Methanol und Wasser enthaltendes Syntheseprodukt (106) entsteht, das in ein Wasserstoff und Kohlenstoffdioxid enthaltendes Recyclegas (102) zur Bildung des Syntheseeinsatzes (103) sowie Methanol und Wasser enthaltendes Rohmethanol (107) getrennt wird, aus dem nachfolgend in einer Methanolaufbereitung ein Methanolprodukt (117) entsteht, wobei der Wasserstoff (101a) in einem zweiten Betriebsmodus mit einem unterhalb des Schwellwerts liegenden Mengenstrom bereitgestellt wird, der nicht ausreicht, um genügend Rohmethanol (107) für die Aufrechterhaltung des Betriebs der Methanolaufbereitung (210) zu produzieren. Kennzeichnend hierbei ist, dass ein Teil (115) des im ersten Betriebsmodus erzeugten Rohmethanols (107) in einen Rohmethanolspeicher (90) eingeleitet wird, aus dem im zweiten Betriebsmodus Rohmethanol entnommen wird, um die produzierte Rohmethanolmenge so weit zu ergänzen, dass Rohmethanol (116) in einer für den Betrieb der Methaulaufbereitung erforderlichen Menge erhalten wird.

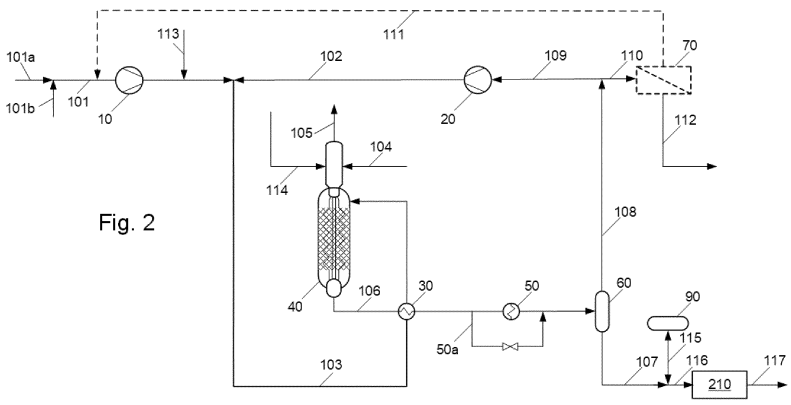


Fig. 2

EP 4 700 005 A1

(52) Gemeinsame Patentklassifikation (CPC): (Forts.)

C-Sets
**C07C 29/151, C07C 31/04**

## Beschreibung

Die Erfindung betrifft ein Verfahren zur Erzeugung von Methanol, bei dem Wasserstoff in einem ersten Betriebsmodus mit einem oberhalb eines Schwellwerts liegenden Mengenstrom bereitgestellt und zusammen mit Kohlenstoffdioxid als Frischeinsatz in einen Synthesekreislauf eingeleitet wird, um einen Wasserstoff und Kohlenstoffdioxid umfassenden Syntheseeinsatz zu bilden, aus dem in einem Methanol-Synthesereaktor ein Wasserstoff, Kohlendioxid, Methanol und Wasser enthaltendes Syntheseprodukt entsteht, das in ein Wasserstoff und Kohlenstoffdioxid enthaltendes Recyclegas zur Bildung des Syntheseeinsatzes sowie Methanol und Wasser enthaltendes Rohmethanol getrennt wird, aus dem nachfolgend in einer Methanolaufbereitung ein Methanolprodukt entsteht, wobei der Wasserstoff in einem zweiten Betriebsmodus mit einem unterhalb des Schwellwerts liegenden Mengenstrom bereitgestellt wird, der nicht ausreicht, um genügend Rohmethanol für die Aufrechterhaltung des Betriebs der Methanolaufbereitung zu produzieren.

Weiterhin betrifft die Erfindung eine Vorrichtung zur Durchführung des erfindungsgemäßen Verfahrens.

Die Methanolerzeugung ist ein Schlüsselprozess in der chemischen Industrie. Methanol stellt eine wichtige Grundchemikalie für die Herstellung von beispielsweise Formaldehyd, Acetaten und Kunststoffen dar und kann zudem als Treibstoffzusatz verwendet werden. Die Methanolsynthese aus einem Wasserstoff und zumindest ein Kohlenstoffoxid enthaltenden Syntheseeinsatz dominiert aufgrund ihrer Effizienz und der breiten Verfügbarkeit von Erdgas als Ausgangsmaterial für die Gewinnung von Wasserstoff und Kohlenstoffoxiden. Alternativ kann auch Kohle oder Biomasse als Rohstoff verwendet werden.

Die wichtigsten Reaktionen bei der Methanolsynthese sind die Methanolbildung und die Konvertierungsreaktion, die in den nachfolgenden Gleichungen angegeben sind:

Methanolbildung:

$$CO + 2\ H_2 \leftrightarrow CH_3OH$$

$$\Delta\ H_R = -\ 90{,}77\ kJ/mol$$

$$CO_2 + 3\ H_2 \leftrightarrow CH_3OH + H_2O$$

$$\Delta\ H_R = -\ 49{,}16\ kJ/mol$$

Konvertierungsreaktion:

$$CO_2 + H_2 \leftrightarrow CO + H_2O$$

$$\Delta\ H_R = -\ 41{,}21\ kJ/mol$$

Im Falle der Nutzung von Erdgas als Ausgangsmaterial wird dieses typischerweise zunächst einer Dampf- oder Autothermalreformierung unterzogen, um ein Wasserstoff Kohlenstoffmonoxid und Kohlenstoffdioxid umfassendes Synthesegas zu erzeugen. Dieser Schritt beinhaltet die Reaktion von Erdgas mit Wasserdampf bzw. Sauerstoff und Wasserdampf bei hohen Temperaturen und unter Verwendung eines Katalysators. Bei der Nutzung von Kohle oder Biomasse kommt oft auch die Partielle Oxidation zum Einsatz.

Das erzeugte Synthesegas wird gewöhnlich einer Aufbereitung und/oder Reinigung unterzogen, ehe es als Frischeinsatz der Methanolsynthese zugeführt wird, wobei es verdichtet, mit einem Wasserstoff sowie Kohlenstoffoxide enthaltenden Recyclegas zum Syntheseeinsatz gemischt und anschließend mit katalytischer Unterstützung in einem Methanol-Synthesereaktor unter hohem Druck (5 bis 10 MPa) und bei moderaten Temperaturen (200 bis 300°C) zu einem Syntheseprodukt umgesetzt wird, das neben Methanol und Wasser auch erhebliche Wasserstoff- und Kohlenstoffoxidanteile enthält. Katalysatoren auf Basis von Kupfer, Zinkoxid und Aluminiumoxid sind hierbei üblich und typischerweise in einem oder mehreren Festbetten angeordnet.

Um Methanol und Wasser durch Kondensation abzuscheiden und Rohmethanol sowie das weitgehend aus Wasserstoff und Kohlestoffoxiden bestehende Recyclegas zu erhalten, wird das Syntheseprodukt abgekühlt. Da abhängig von der Synthesegaszusammensetzung, der Art des eingesetzten Katalysators und der gewählten Prozessparameter in einem Reaktordurchgang lediglich Kohlenstoffumsätze zwischen 50 und 80% erreicht werden, wird ein Synthesekreislauf etabliert, in dem das Recyclegas zurückgeführt und bei der Bildung des Syntheseeinsatzes verwendet wird. Um die Anreicherung von Inerten im Synthesekreislauf zu verhindern, ist es üblich, einen kleinen Teil des Recyclegases als Purgegas auszuschleusen.

Die Zusammensetzung des der Methanolsynthese zugeführten Syntheseeinsatzes wird durch eine als Stöchiometriezahl (SN) bezeichnete Kenngröße charakterisiert, zu deren Berechnung die molaren Konzentrationen der an der Synthesereaktion teilnehmenden Komponenten Wasserstoff (H2), Kohlendioxid (CO2) und Kohlenmonoxid (CO) gemäß

$$SN = ([H_2] - [CO_2])/([CO] + [CO_2])$$

ins Verhältnis gesetzt werden. Synthesegaszusammensetzungen mit SN = 2 sind stöchiometrische Mischungen, die zumindest theoretisch einen vollständigen Umsatz zu Methanol erlauben. Werte für SN > 2 entsprechen einem Wasserstoffüberschuss in der Methanolsynthese. Werte für SN < 2 entsprechen einem Wasserstoffdefizit, bei dem nicht sämtliche Kohlenstoffoxide zu Methanol umgesetzt werden können.

In einer der Synthese nachgeschalteten Methanolaufbereitung wird aus dem Rohmethanol destillativ reines Methanol abgetrennt. Dieser Schritt kann mehrstufig durchgeführt werden, um die gewünschte Reinheit zu erreichen bzw. den Energieaufwand zu minimieren. Das reine Methanol wird gelagert und entweder direkt als Produkt verkauft oder als Ausgangsstoff für die Herstellung anderer Chemikalien verwendet.

Zukünftige Entwicklungen werden zunehmend auf erneuerbare Rohstoffe und nachhaltigere Produktionsmethoden abzielen. So können beispielsweise dem Synthesekreislauf Elektrolysewasserstoff sowie aus Abgasen zurückgewonnenes Kohlenstoffdioxid, getrennt oder gemischt als Frischgase zugeführt werden. Wird die für die Elektrolyse benötigte elektrischer Leistung aus regenerativen Quellen wie Wind- oder Solarkraftwerken, oder als Überschuss aus dem öffentlichen Netz bezogen, steht sie nicht konstant zur Verfügung und kann zeitweise so weit absinken, dass die erzeugbare Wasserstoffmenge nicht für einen Betrieb der Methanolsynthese ausreicht, so dass die Methanolproduktion unterbrochen werden muss.

Ein Elektrolyseur lässt sich relativ einfach und schnell an wechselnde Einsatzbedingungen anpassen, weshalb die Wasserstoffproduktion in erster Näherung proportional zur zugeführten elektrischen Leistung ist und sowohl der Mengenstrom des im Elektrolyseur produzierten Wasserstoffs als auch der Mengenstrom des Syntheseeinsatzes mit der Höhe der verfügbaren elektrischen Leistung schwanken.

Im Vergleich zu einem Elektrolyseur, können der Metahol-Synthesereaktor sowie die weiter stromabwärts angeordnete Methanolaufbereitung nur langsamer und in wesentlich engeren Grenzen an schwankende Betriebsbedingungen angepasst werden. Zwar kann der Normalbetrieb des Methanol-Synthesereaktors auch noch mit 30% seiner Nennlast aufrechterhalten werden, jedoch muss die gesamte Anlage bereits bei einer höheren Last abgeschaltet werden, weil die Teillastfähigkeit der Methanolaufbereitung - in erster Linie durch die Mindestberieselungsdichte der Böden in der dort eingesetzten Destillationskolonne - auf ca. 50% begrenzt ist.

Um ein Abschalten der Anlage zu vermeiden, kann in Zeiten, in denen ausreichend elektrische Leistung zur Verfügung steht, mehr Wasserstoff produziert werden, als in der Methanolsynthese eingesetzt werden kann. Der überschüssig erzeugte Wasserstoff wird gespeichert und in Zeiten mangelnder elektrischer Leistung dazu verwendet, das Frischgas bzw. den Syntheseeinsatz mit einem Mengenstrom zu bilden, der einen Anlagenbetrieb mit wenigstens 50% der Nennlast erlaubt.

Der Wasserstoff wird gasförmig, adsorptiv bzw. absorptiv gebunden oder tiefkalt in flüssiger Form gespeichert. In jedem Fall ist die Speicherung kostenintensiv, da die erforderlichen Speicher sehr groß und/oder druckfest oder wärmeisoliert ausgeführt sein müssen und darüber hinaus Aufwendungen für Apparate und Betriebsmittel zur Verdichtung bzw. Abkühlung des Wasserstoffs in erheblichem Umfang anfallen. Zudem besteht die Gefahr, dass die gespeicherte Wasserstoffmenge zur Überbrückung eines länger andauernden Mangels an elektrischer Leistung nicht ausreicht und die Methanolsynthese trotz allem abgeschaltet werden muss.

Außerplanmäßiges Ab- und Anfahren der Metaholsynthese sollte jedoch möglichst vermieden werden, da die damit verbundenen Druck- und Temperaturwechsel nicht nur den Synthesereaktor selbst, sondern auch Hilfsapparate, wie etwa Verdichter, mechanisch stark beanspruchen. Der im Synthesereaktor eingesetzte Katalysator kann zerfallen, wodurch seine Aktivität abnimmt, der Druckverlust über das Katalysatorbett ansteigt und die Gase im Synthesereaktor fehlverteilt werden. Darüber hinaus kann während des mehrere Tage dauernden Wiederanfahrens der Metaholsynthese kein Methanol mit einer für die Abgabe als Produkt ausreichenden Qualität erzeugt werden, auch wenn Wasserstoff in dieser Zeit bereits in ausreichender Menge zur Verfügung steht.

Aufgabe der vorliegenden Erfindung ist es daher, ein Verfahren sowie eine Vorrichtung der gattungsgemäßen Art anzugeben, mit denen die Nachteile des Standes der Technik überwunden werden.

Zur Lösung der gestellten Aufgabe schlagen die hier präsentierten Konzepte vor, den Synthesekreislauf von den

nachfolgenden Prozessen, insbesondere der Methanolaufbereitung zu entkoppeln. Die vorgestellten Lösungen umfassen Ansätze für den Betrieb des Synthesekreislaufs bei Teillast oder in Standby, kombiniert mit einem herkömmlichen Teillastbetrieb der nachfolgenden Prozesse. Auf diese Weise werden die Belastungseinschränkungen der Reinigung (Mindestberieselungsdichte) nicht auf den Synthesekreislauf übertragen.

Um dies zu erreichen, wird ein Teil des im ersten Betriebsmodus erzeugten Rohmethanols in einen Rohmethanolspeicher eingeleitet, aus dem im zweiten Betriebsmodus Rohmethanol entnommen wird, um die produzierte Rohmethanolmenge so weit zu ergänzen, dass Rohmethanol in einer für den Betrieb der Methaulaufbereitung erforderlichen Menge erhalten wird.

Das erfindungsgemäße Verfahren ermöglicht es, den Methanol-Synthesereaktor mit einer Teillast zu betreiben, die kleiner ist als die minimale Teillast der Methanolaufbereitung, ohne dass die Methanolaufbereitung abgeschaltet und die Methanolproduktion unterbrochen werden muss. In letzter Konsequenz ist es sogar möglich, die Methanolsynthese völlig einzustellen und gleichzeitig die Methanolaufbereitung weiter zu betreiben.

Nach dem Stand der Technik kann der Methanol-Synthesereaktor nur bis zu einer kritischen Teillast, die typischerweise bei ca. 30% der Volllast liegt, betrieben werden, so dass es nicht möglich ist, die sich durch die erfindungsgemäße Entkopplung des Synthesekreislaufs von den nachfolgenden Prozessen ergebenden Möglichkeiten voll auszuschöpfen.

Zur Überwindung dieses Nachteils wird vorgeschlagen, die Stöchiometriezahl des Syntheseeinsatzes gegenüber dem Normalbetrieb anzuheben, sobald der Mengenstrom des Wasserstofffrischeinsatzes nicht ausreicht, um den Methanol-Synthesereaktor mit einer größeren als der kritischen Teillast zu betreiben. Auf diese Weise kann das Absinken des Reaktordrucks begrenzt werden.

Vorzugsweise wird die Stöchiometriezahl dadurch erhöht, dass die als Frischgas zugeführte Kohlendioxidmenge im Vergleich zur Wasserstoffmenge überproportional reduziert wird. Auf diese Weise kann die Einspeisung von Kohlenstoffdioxid völlig unterbunden werden, so dass die Methanolsynthesereaktion zum Stillstand kommt.

Alternativ oder zusätzlich kann der Mengenstrom des Recyclegases reduziert werden. Hierdurch verringert sich der Stofffluss durch den Methanol-Synthesereaktor stärker, als es allein aufgrund der reduzierten Menge an Frischgas der Fall wäre. Da die Reaktionsrate, die den Anteil des Syntheseeinsatzes angibt, der bei einem einmaligen Durchlauf des Methanol-Synthesereaktors zu Methanol umgesetzt wird, durch das druckabhängige Reaktionsgleichgewicht limitiert ist, kann sie sich nicht ausreichend erhöhen, um die reduzierte Menge des Syntheseeinsatzes auszugleichen. Folglich wird die Reaktionsrate herabgesetzt, so dass bei einer Verringerung der Anlagenlast der Druck im Synthesekreislauf weniger stark abnimmt als ohne eine Reduzierung des Mengenstroms des Recyclegases, bzw. konstant bleibt oder sich sogar erhöht.

Der Mengenstrom des Recyclegases kann beispielsweise über ein einstellbares Drosselorgan und/oder einen Verdichter mit veränderbarer Förderleistung kontrolliert werden.

Weiterhin kann alternativ dem Rohmethanolspeicher Rohmethanol entnommen, um das enthaltene Methanol in einer Spalteirichtung in Wasserstoff und Kohlendioxid zu spalten und ein Synthesegas zu erhalten, mit dem die Frischgase so ergänzt werden, dass der Methanol-Synthesereaktor mit einer größeren als der kritischen Teillast betrieben werden kann. Das hierbei erzeugte Rohmethanol kann wieder dem Rohmethanolspeicher zugeführt werden.

Ein Teil des in dem zweiten Betriebsmodus dem Rohmethanolspeicher entnommenen Methanols kann auch zur Bereitstellung von Reaktionswärme für die Methanolspaltung verbrannt werden. Auch der Einsatz von Dampf und/oder elektrischer Energie zur Methanolspaltung ist alternativ oder zusätzlich möglich.

In bestimmten Ausgestaltungen wird die Methanolsynthese mit einem als isothermer Dampferzeuger ausgeführten Methanol-Synthesereaktor betrieben, dessen Temperatur mittels einer Dampfdruckregelung kontrolliert wird. Durch entsprechende Maßnahmen kann eine besonders vorteilhafte Temperatureinstellung im Synthesekreislauf realisiert werden.

In bestimmten Ausgestaltungen wird die Methanolsynthese in einem isothermen Synthesereaktor mit gewickelten, von Katalysatormaterial umgebenen Rohren durchgeführt, wobei das Synthesegas über das Katalysatormaterial geführt wird und die gewickelten Rohre von Kühlmedium durchströmt werden. Dies ermöglicht eine besonders einfache Anpassung der spezifischen Wärmeübertragungsfläche.

In bestimmten Ausgestaltungen wird in dem zweiten Betriebsmodus eine Dampfeinspeisung in das Verfahren vorgenommen. Auf diese Weise ist es möglich, den Synthesekreislauf auch bei einem Ausbleiben der Wasserstoffeinspeisung auf einer geeigneten Temperatur für einen raschen Neustart zu halten.

In bestimmten Ausgestaltungen kann Wärme dem Syntheseprodukt mittels eines Wärmetauschers entzogen werden, wobei der Wärmetauscher in dem zweiten Betriebsmodus zumindest teilweise mittels eines Bypasses umgangen wird, so dass die Wärme im Wesentlichen im Synthesekreislauf verbleibt.

In dem zweiten Betriebsmodus kann ein Inertgas in den um die Methanolsynthese gebildeten Synthesekreislauf eingespeist werden, um eine entsprechend verringerte Gasmenge zu kompensieren.

Weiterhin wird ein Anlage zur Herstellung von Methanol vorgeschlagen, umfassend einen Methanol-Synthesereaktor, der dafür eingerichtet ist, Wasserstoff sowie einen oder mehrere Kohlenstoffoxide einer Methanolsynthese zu

unterwerfen und ein Syntheseprodukt abzuführen, das in der Methanolsynthese nicht umgesetzte Anteile des Wasserstoffs und des einen oder der mehreren Kohlenstoffoxide sowie Methanol und Wasser enthält, eine Trenneinrichtung, mit der Methanol und Wasser aus dem Syntheseprodukt in Methanol und Wasser enthaltendes Rohmethanol überführt werden können, sowie eine mit der Trenneinrichtung verbundene Methanolaufbereitung zur Erzeugung eines Methanolprodukts aus Rohmethanol.

Anlagenseitig wird die gestellte Aufgabe dadurch gelöst, dass sie einen Rohmethanolspeicher aufweist, in den in einem ersten Betriebsmodus in der Trenneinrichtung erhaltenes Rohmethanol eingeleitet und aus dem in einem zweiten Betriebsmodus Rohmethanol zur Weiterleitung in die Methanolaufbereitung entnommen werden kann.

Zu weiteren Merkmalen und Vorteilen einer entsprechenden Anlage und Ausgestaltungen hiervon sei auf die obigen Erläuterungen betreffend das vorgeschlagene Verfahren und seine Ausgestaltungen ausdrücklich verwiesen, da diese hierfür in gleicher Weise gelten.

Zeichnungen

Die Erfindung wird nachfolgend anhand dreier, in den **Figuren 1** bis **3** schematisch dargestellter Ausführungsbeispiele näher erläutert.

In der Figur 1 ist eine klassische Methanolsynthese in stark vereinfachter Weise veranschaulicht, während die Figuren 2 und 3 Ausgestaltungen der Erfindung zeigen. In den Figuren sind gleiche Anlagenteile und Stoffströme mit gleichen Bezugszeichen markiert.

Dem Synthesekreislauf der in Figur 1 veranschaulichten Methanolsynthese, kann Synthesegas als Makeup bzw. Frischeinsatz 101 zugeführt werden. In einem Einsatzverdichter 10 erfolgt eine Verdichtung, beispielsweise auf die eingangs erwähnten Drücke. Der Synthesekreislauf ist für eine Methanolsynthese in an sich üblicher Weise ausgebildet. Der Frischeinsatz 101 wird dabei etwa durch Dampfreformierung, autotherme Reformierung oder partielle Oxidation aus fossilen Rohstoffen in einem kontinuierlichen Prozess gewonnen. In diesem Fall enthält der Frischeinsatz 101 die Hauptkomponenten Wasserstoff, Kohlenstoffmonoxid und Kohlenstoffdioxid sowie Nebenkomponenten wie Methan oder Stickstoff, die sich bei der Methanolsynthese inert verhalten.

Durch Vereinigung des Frischeinsatzes 101 mit Recyclegas 102 aus der Recycleverdichtung 20, wird ein Syntheseeinsatz 103 gebildet, der nach Anwärmung in einem Feed-Effluent-Wärmetauscher 30 dem Methanol-Synthesereaktor 40 zugeführt wird, um mit katalytischer Unterstützung in einer exothermen Reaktion zu einem Methanol enthaltenden Syntheseprodukt 106 umgesetzt zu werden. Bei der Umsetzung anfallende Abwärme kann beispielsweise zur Erzeugung von Sattdampf 105 aus Kesselspeisewasser 104 genutzt werden. Das heiß aus dem Methanol-Synthesereaktor 40 austretende Syntheseprodukt 106 wird im Feed-Effluent-Wärmetauscher 30 gegen den Syntheseeinsatz 103 und weiter im Wärmetauscher 50 gegen Kühlwasser abgekühlt, um Methanol zusammen mit Wasser auszukondensieren und ein zweiphasiges Stoffgemisch zu erhalten, das im Abscheider 60 in ein Wasserstoff und Kohlenstoffoxide enthaltendes Restgas 108 sowie wasserhaltiges Rohmethanol 107 getrennt wird, aus dem in der Methanolaufbereitung 210 das Methanolprodukt 117 entsteht.

Abhängig von der Zusammensetzung des Syntheseeinsatzes 103, den Prozessbedingungen, dem eingesetzten Katalysator und dem gewählten Verfahren, kann in einem einzigen Durchgang durch den Methanol-Synthesereaktor 40 aufgrund thermodynamischer Limitierungen lediglich eine Kohlenstoffumwandlung von ca. 50% bis 80% erreicht werden. Daher wird das Restgas 108 nach Ausleitung eines Purge- oder Spülgasstroms 110 über Leitung 109 dem Recycleverdichter 20 zugeführt, der es zu zum Recyclegas 102 verdichtet und zur Steigerung der Wasserstoff- und Kohlenstoffumwandlung vor den Methanol-Synthesereaktor 40 zurückführt.

Der Methanol-Synthesereaktor 40 kann in fachüblicher Weise als Einzelreaktor oder Reaktorsystem ausgebildet sein, beispielsweise umfassend adiabatische Teilreaktoren mit Zwischenkühlern, quenchgekühlte (Teil-)Reaktoren, gasgekühlte (Teil-)Reaktoren und isotherme, dampfproduzierende (Teil-)Reaktoren in beliebiger serieller und/oder paralleler Anordnung. Ausgestaltungen der Erfindung sind durch die Art des oder der Methanol-Synthesereaktor in keiner Weise beschränkt.

Die Größe des Spülgasstroms 110 hängt von der Konzentration der Inerten, den Prozessbedingungen, dem Katalysator, dem Reaktortyp und der in üblicher Weise definierten Stöchiometriezahl ab. Insbesondere soll durch die Ausleitung des Spülgasstroms 110 verhindert werden, dass sich Inerte im Synthesekreislauf anreichern.

Wenn Wasserstoff benötigt wird, um die Zusammensetzung des Syntheseeinsatzes 103 auf die erforderliche Stöchiometriezahl einzustellen, kann er mit verschiedenen Methoden etwa aus dem Spülgasstrom 110 abgetrennt werden. Beispielsweise ist es möglich, den Spülgasstrom 110 über eine Membrantrennstufe 70 in einen Wasserstoffrecycle 111 und ein Restgas 112 zu trennen.

Ein klassischer Metholsyntheseprozess wie der in Figur 1 dargestellte, wird mit weitgehend konstanter Last und nur geringen Laständerungsgradienten betrieben.

Das Ausführungsbeispiel der Figur 2 zeigt eine Methanolsynthese, bei der zur Bildung des Frischeinsatzes 101 Wasserstoff 101a eingesetzt wird, der unter Verwendung von Strom aus erneuerbaren Energiequellen (wie Sonne

und/oder Wind) durch Elektrolyse erzeugt und mit stark schwankendem Mengenstrom zur Verfügung gestellt wird. Das ebenfalls zur Bildung des Frischeinsatzes 101 zugeführte Kohlendioxid 101b wird beispielsweise aus einem Rauchgas abgetrennt.

Der Methanol-Synthesereaktor 40 kann gewöhnlich auch noch mit 30% seiner Nennlast betrieben werden, jedoch ist die Teillastfähigkeit der Methanolaufbereitung - in erster Linie durch die Mindestberieselungsdichte der Böden in der dort eingesetzten Destillationskolonne - auf ca. 50% begrenzt ist. Steht der Wasserstoff 101a in einer oberhalb eines Schwellwerts liegenden Menge zur Verfügung, die es erlaubt, Rohmethanol 107 mit mehr als 50% der für dir Nennlast benötigten Menge zu erzeugen, wird die Methanol-Synthese zwar in Teillast aber in einem weitgehend dem Normalbetrieb entsprechenden ersten Betriebsmodus betrieben. Zumindest dann, wenn der Methanol-Synthesereaktor 40 mit seiner Nennlast betrieben wird, kann im ersten Betriebsmodus ein Teil 115 des erzeugten Rohmethanols 107 abgezweigt und in einen Rohmethanolspeicher 90 eingeleitet und nur der verbleibende Rest 116 in die Methanolaufbereitung 210 weitergeführt werden.

In einem zweiten Betriebsmodus wird der Wasserstoff 101a mit einem unterhalb des Schwellwerts liegenden Mengenstrom zur Verfügung gestellt und reicht daher nicht dazu aus, genügend Rohmethanol 107 für die Aufrechterhaltung des Betriebs der Methanolaufbereitung 210 zu produzieren. Um ein Abschalten der Methanolaufbereitung 210 zu vermeiden, wird die Menge des frisch produzierten Rohmethanols 107 durch Rohmethanol ergänzt, das über Leitung 115 aus dem Rohmethanolspeicher 90 entnommen wird.

Steht weniger als 30% der für die Nennlast benötigten Wasserstoffmenge zur Verfügung, müssen nach dem Stand der Technik der Methanol-Synthesereaktor 40 abgeschaltet und die Methanolproduktion eingestellt werden. Um zumindest die Abschaltung des Methanol-Synthesereaktor 40 vermeiden zu können, wird erfindungsgemäß vorgeschlagen, die Stöchiometriezahl des Syntheseeinsatz 103 zu erhöhen, um den Druckabfall im Synthesekreislauf zu begrenzen.

Ferner können eine Inertgaseinspeisung 113 sowie eine Dampfeinspeisung 114 zum Warmhalten des Methanol-Synthesereaktor 40 vorgenommen werden. Weiterhin ist es möglich, die Menge des Recyclegases 102 über die Recycleverdichtung 20 auf einen für die verwendeten Apparate tolerablen Minimalwert zu reduzieren. Ein Abfluss des Spülgases 110 kann über ein Ventil begrenzt oder verhindert werden; der Wärmetauscher 50 kann über einen Bypass 50a umgangen werden.

Durch diese Maßnahmen kann die Teillastfähigkeit des Methanol-Synthesereaktors 40 bis zu einem heißen Standbybetrieb, der ohne Wasserstoffverbrauch abläuft, erweitert werden.

Vorzugsweise handelt es sich bei dem Methanol-Synthesereaktor 40 um einen Isothermreaktor mit einem gewickelten Dampferzeuger, der eine einfache Regelung auf eine konstante Betriebstemperatur durch die Steuerung des Dampfdrucks erlaubt. Wärmeverluste des Synthesekreislaufs bei niedriger Teillast oder im heißen Standbybetrieb können durch die Zufuhr von Wärme über die Dampfeinspeisung 114, insbesondere unter Verwendung von Hochdruckdampf bei 30 bis 50 bar, oder alternativ über eine elektrische Heizung ausgeglichen werden. Dadurch kann die Temperatur des Methanol-Synthesereaktors 40 auf einem isothermen Niveau gehalten werden (z.B. zwischen 200 und 270°C), so dass er ohne weiteres Vorheizen jederzeit startbereit ist.

Bei Lastschwankungen und im Standbybetrieb wird der Katalysator nur sehr geringfügig thermisch belastet, so dass sehr schnelle Lastwechselgradienten von über 60% pro Stunde möglich sind. Darüber hinaus ermöglicht ein isothermer Methanol-Synthesereaktor 40 mit gewickeltem Dampferzeuger und dem Katalysator auf der Mantelseite eine einfache Anpassung der spezifischen Wärmeübertragungsfläche im Vergleich zu klassischen Reaktoren mit geraden Dampferzeugerrohren.

Um die Anlage im heißen Standby zu betreiben, kann die Zufuhr des Kohlenstoffdioxids 101b vollständig eingestellt werden. Dadurch wird die Methanolbildung gestoppt, nachdem das verbleibende Kohlenstoffmonoxid bzw. Kohlenstoffdioxid in der Syntheseschleife bis zur Gleichgewichtskonzentration verbraucht ist. Der Spülgasstrom 110 aus dem Kreislauf wird hierbei insbesondere vollständig blockiert, und die Recycleverdichtung 20 führt das verbleibende Synthesegas mit z.B. 50% des Nennlastdurchflusses zurück.

Gasverluste aus dem Kreislauf können für diesen heißen Standbybetrieb durch Einspeisung von Wasserstoff, z.B. aus einen Wasserstoffspeicher (nicht dargestellt), in den Synthesekreislauf ausgeglichen werden, so dass der Druck weitgehend konstant bleibt. Dadurch steigt das Rückführungsverhältnis im Kreislauf auf sehr hohe Werte an, bis das gesamte Kohlenstoffdioxid verbraucht ist und nur noch reiner Wasserstoff (und Inertgase) zurückgeführt werden. Alternativ kann die Druckhaltung bei Bedarf auch über eine Inertgaseinspeisung 113 (beispielsweise von Stickstoff, Argon, Methan oder Helium) erfolgen.

Eine weitere Ausgestaltung der hier vorgeschlagenen Erfindung ist in Figur 3 dargestellt.

Wie in Figur 3 veranschaulicht, kann bei Bedarf der fehlende Frischeinsatz 101 zur Aufrechterhaltung der Mindestlast des Synthesekreislaufs in einem kleinen Methanol-Reformer 220 erzeugt werden. Dazu wird eine kleine Menge Rohmethanol aus dem Rohmethanolspeicher 90 entnommen, mittels einer Pumpe 230 auf einen sinnvollen Druck gebracht (z.B. auf 2,5 MPa), verdampft, erhitzt und in einem katalytischen, endothermen Prozess in ein hauptsächlich Wasserstoff, Kohlenstoffmonoxid und Kohlenstoffdioxid enthaltendes Synthesegas umgewandelt. Dieses Gasgemisch,

hier mit 118 bezeichnet, kann als Nachschub für den Synthesekreislauf verwendet werden.

Die für die Reformierung erforderliche Reaktionswärme kann durch die Verbrennung von Methanol, durch elektrische Heizung oder Dampf bereitgestellt werden.

## Patentansprüche

1. Verfahren zur Erzeugung von Methanol, bei dem Wasserstoff (101a) in einem ersten Betriebsmodus mit einem oberhalb eines Schwellwerts liegenden Mengenstrom bereitgestellt und zusammen mit Kohlenstoffdioxid (101b) als Frischeinsatz (101) in einen Synthesekreislauf eingeleitet wird, um einen Wasserstoff und Kohlenstoffdioxid umfassenden Syntheseeinsatz (103) zu bilden, aus dem in einem Methanol-Synthesereaktor (40) ein Wasserstoff, Kohlendioxid, Methanol und Wasser enthaltendes Syntheseprodukt (106) entsteht, das in ein Wasserstoff und Kohlenstoffdioxid enthaltendes Recyclegas (102) zur Bildung des Syntheseeinsatzes (103) sowie Methanol und Wasser enthaltendes Rohmethanol (107) getrennt wird, aus dem nachfolgend in einer Methanolaufbereitung ein Methanolprodukt (117) entsteht, wobei der Wasserstoff (101a) in einem zweiten Betriebsmodus mit einem unterhalb des Schwellwerts liegenden Mengenstrom bereitgestellt wird, der nicht ausreicht, um genügend Rohmethanol (107) für die Aufrechterhaltung des Betriebs der Methanolaufbereitung (210) zu produzieren, **dadurch gekennzeichnet, dass** ein Teil (115) des im ersten Betriebsmodus erzeugten Rohmethanols (107) in einen Rohmethanolspeicher (90) eingeleitet wird, aus dem im zweiten Betriebsmodus Rohmethanol entnommen wird, um die produzierte Rohmethanolmenge so weit zu ergänzen, dass Rohmethanol (116) in einer für den Betrieb der Methaulaufbereitung erforderlichen Menge erhalten wird.

2. Verfahren nach Anspruch 1, **dadurch gekennzeichnet, dass** das Recyclegas (102) im zweiten Betriebsmodus mit einem geringen Mengenstrom als im ersten Betriebsmodus zur Bildung des Syntheseeinsatzes zurückgeführt wird.

3. Verfahren nach einem der Ansprüche 1 oder 2, **dadurch gekennzeichnet, dass** ein als Dampferzeuger ausgeführter Methanol-Synthesereaktor (40) eingesetzt wird, dessen Leistung mittels einer Dampfdruckregelung eingestellt wird.

4. Verfahren nach Anspruch 3, **dadurch gekennzeichnet, dass** dem Methanol-Synthesereaktor (40) im zweiten Betriebsmodus über eine Dampfeinspeisung (114) Wärme zugeführt wird.

5. Verfahren nach einem der Ansprüche 1 bis 4, **dadurch gekennzeichnet, dass** die Stöchiometriezahl des Syntheseeinsatzes (101) im Vergleich zum ersten Betriebsmodus erhöht wird.

6. Verfahren nach einem der Ansprüche 1 bis 5, **dadurch gekennzeichnet, dass** der Syntheseeinsatz (102) im zweiten Betriebsmodus unter Verwendung eines Inertgases (113) gebildet wird.

7. Verfahren nach einem der vorstehenden Ansprüche, bei dem Wärme dem Syntheseprodukt (106) mittels eines Wärmetauschers (50) entzogen wird, wobei der Wärmetauscher (50) in dem zweiten Betriebsmodus zumindest teilweise mittels eines Bypasses (50a) umgangen wird.

8. Verfahren nach einem der vorstehenden Ansprüche, bei dem das im zweiten Betriebsmodus dem Methanolspeicher (90) entnommene Methanol (115) oder ein Teil hiervon einer Methanolspaltung (220) unter Erhalt eines Synthesegases (118) unterworfen und das Synthesegas (118) oder ein Teil hiervon dem Methanol-Synthesereaktor (40) zugeführt wird.

9. Verfahren nach Anspruch 8, bei dem ein Teil des in dem zweiten Betriebsmodus dem Methanolspeicher (90) entnommenen Methanols (115) und/oder Dampf und/oder elektrische Energie zur Bereitstellung von Reaktionswärme für die Methanolspaltung (220) verwendet wird.

10. Anlage zur Herstellung von Methanol, umfassend einen Methanol-Synthesereaktor (40), der dafür eingerichtet ist, Wasserstoff (101a) sowie einen oder mehrere Kohlenstoffoxide (101b) einer Methanolsynthese zu unterwerfen und ein Syntheseprodukt (106) abzuführen, das in der Methanolsynthese nicht umgesetzte Anteile des Wasserstoffs und des einen oder der mehreren Kohlenstoffoxide sowie Methanol und Wasser enthält, eine Trenneinrichtung (60), mit der Methanol und Wasser aus dem Syntheseprodukt (106) in Methanol und Wasser enthaltendes Rohmethanol (107) überführt werden können, sowie eine mit der Trenneinrichtung (60) verbundene Methanolaufbereitung (210) zur Erzeugung eines Methanolprodukts (117) aus Rohmethanol (107), **dadurch gekennzeichnet, dass** sie einen Rohmethanolspeicher (90) aufweist, in den in einem ersten Betriebsmodus in der Trenneinrichtung (60) erhaltenes

Rohmethanol (115) eingeleitet und aus dem in einem zweiten Betriebsmodus Rohmethanol (115) zur Weiterleitung in die Methanolaufbereitung (210) entnommen werden kann.

**11.** Anlage nach Anspruch 10, die zur Durchführung eines Verfahrens nach einem der Ansprüche 1 bis 9 eingerichtet ist.

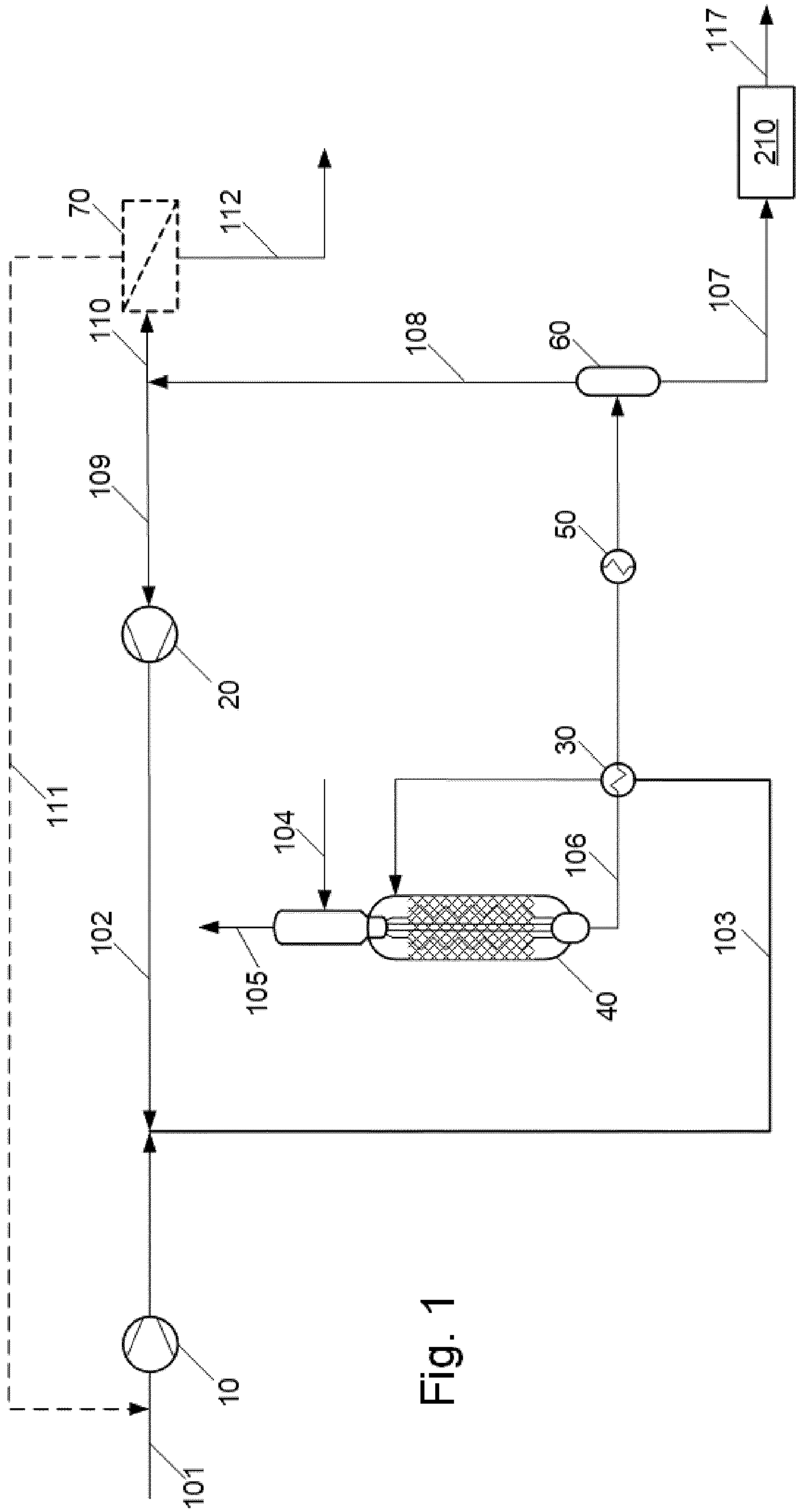
10
20
30
40
50
60
70
101
102
103
104
105
106
107
108
109
110
111
112
117
210

Fig. 1

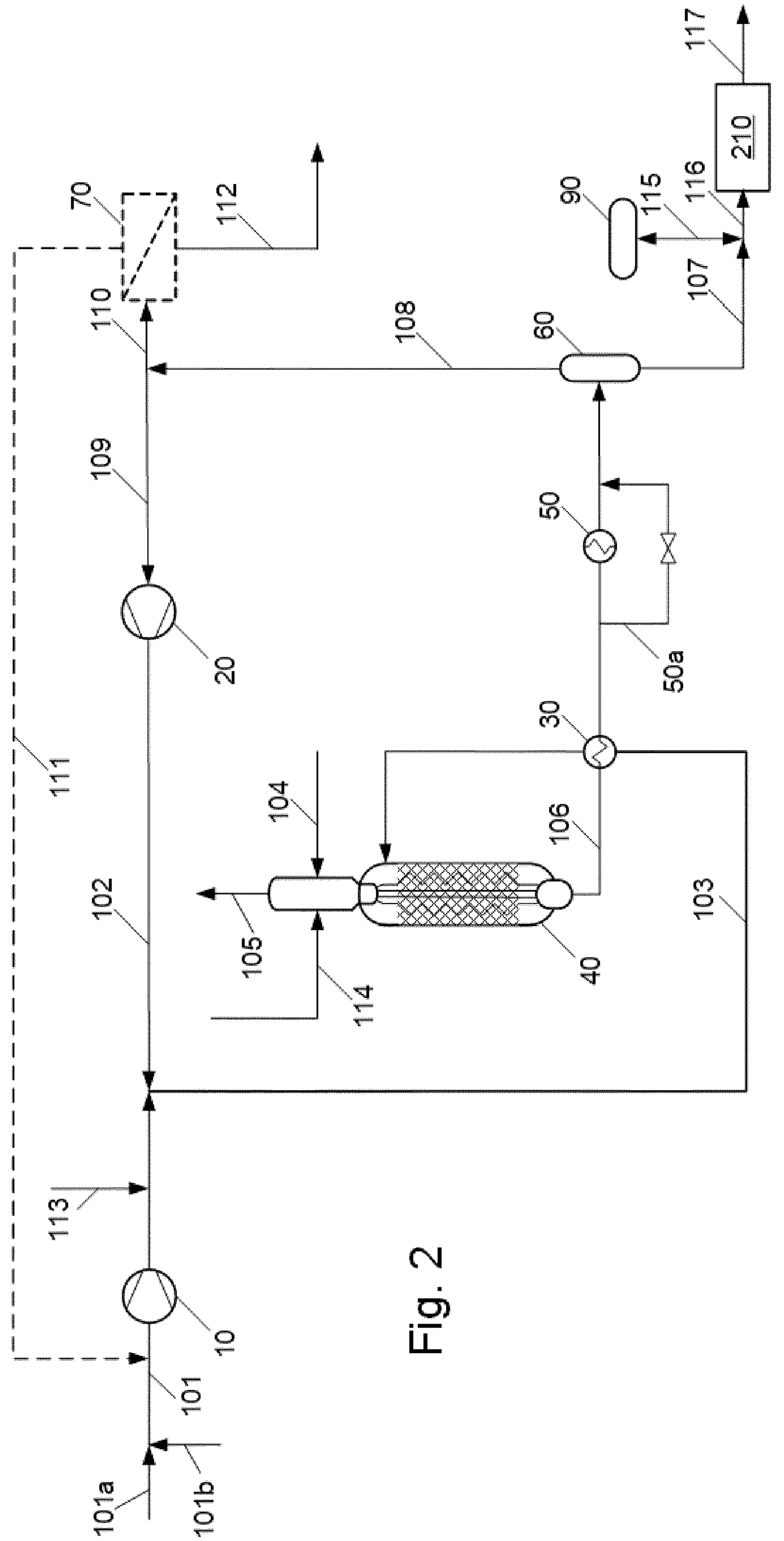
10
20
30
40
50
50a
60
70
90
101
101a
101b
102
103
104
105
106
107
108
109
110
111
112
113
114
115
116
117
210

Fig. 2

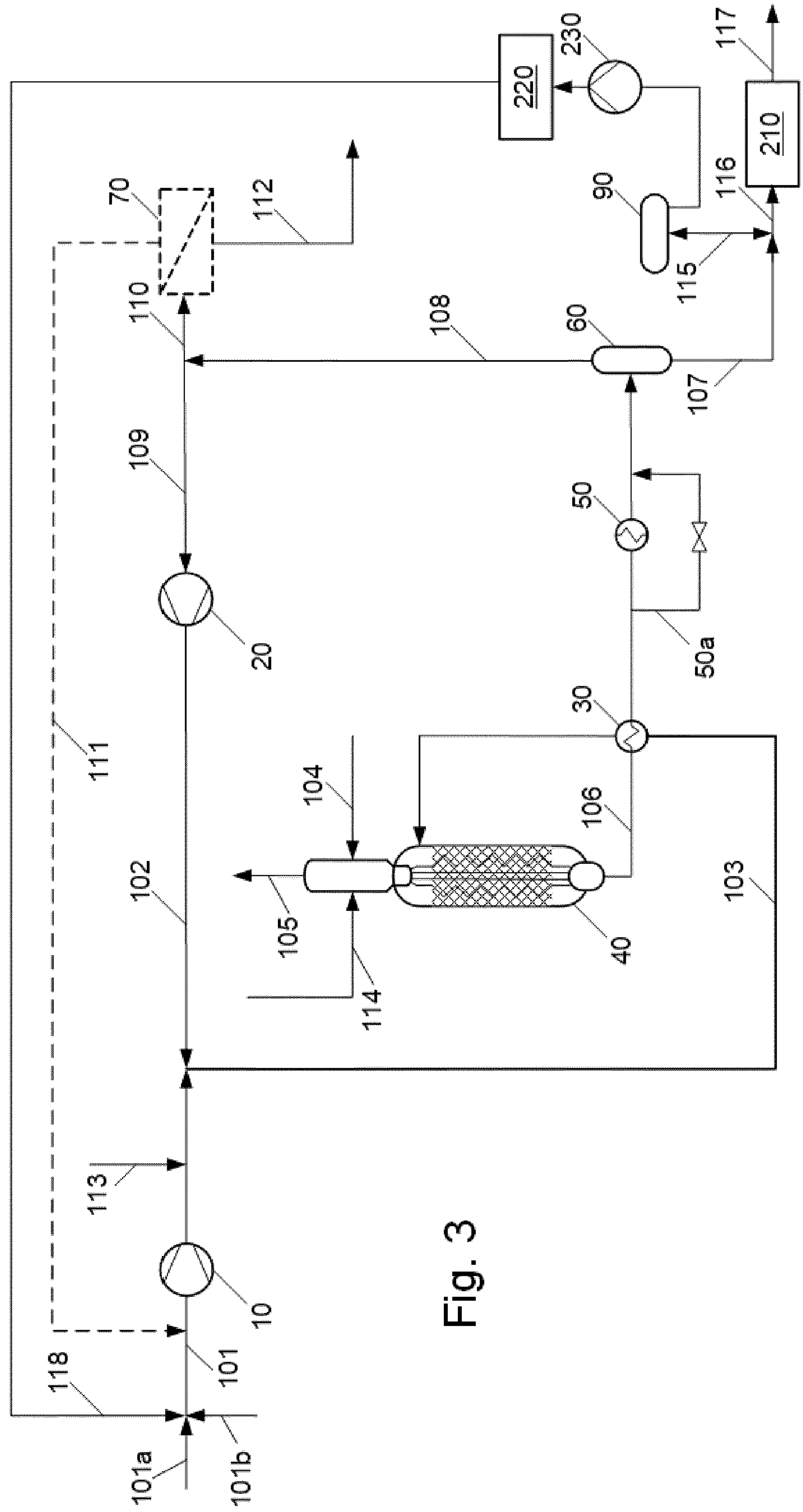
220
230
117
210
116
115
90
70
112
110
108
60
107
109
50
50a
20
30
111
104
106
102
105
103
114
40
113
10
118
101
101a
101b

Fig. 3

Europäisches Patentamt
European Patent Office
Office européen des brevets

# EUROPÄISCHER RECHERCHENBERICHT

Nummer der Anmeldung
EP 24 02 0273

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2023/247316 A1 (TOPSOE AS [DK]) 28. Dezember 2023 (2023-12-28) * Verfahren zur Erzeugung von Methanol aus Synthesegas mittels einer Reaktoreinheit und einer Aufarbeitungseinheit, und wobei die Aufarbeitungseinheit einen Rohmethanolspeicher verwendet: siehe Seite 22, Zeilen 4-17; Seite 17, Zeilen 8-11 * * Der Zweck der Rohmethanol-Aufbewahrung ist als Puffer für nachfolgende Anlageeinheiten zu wirken bei zwischenzeitlichen Strom-Angebots-Schwankungen, die relevant sind für den durch Hydrolyse produzierten Wasserstoff im Synthesegas-Herstellungsverfahren: siehe Seite 22, Zeile9-17 * ----- | 1-11 | INV. C07C29/151 |
| A | EP 3 872 029 A1 (LINDE GMBH [DE]) 1. September 2021 (2021-09-01) * Verfahren zur Herstellung von Methanol aus Synthesegas gewonnen aus Erdgas und Kohlendioxid, das einer Reformierung unterworfen wird (Paragraph [23], [29]), mit zwei Betriebsmodi (siehe Paragraphen [27] und [33] * ----- | 1-11 | |
| | | | RECHERCHIERTE SACHGEBIETE (IPC) |
| | | | C07C |

Der vorliegende Recherchenbericht wurde für alle Patentansprüche erstellt

1

| Recherchenort | Abschlußdatum der Recherche | Prüfer |
|---|---|---|
| Den Haag | 31. Januar 2025 | Lange, Tim |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 24 02 0273

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

31-01-2025

| Im Recherchenbericht angeführtes Patentdokument | Datum der Veröffentlichung | Mitglied(er) der Patentfamilie | Datum der Veröffentlichung |
|---|---|---|---|
| WO 2023247316 A1 | 28-12-2023 | AU 2023287380 A1 | 12-12-2024 |
| | | WO 2023247316 A1 | 28-12-2023 |
| EP 3872029 A1 | 01-09-2021 | KEINE | |

EPO FORM P0461

Für nähere Einzelheiten zu diesem Anhang : siehe Amtsblatt des Europäischen Patentamts, Nr.12/82